(19) **Europäisches Patentamt / European Patent Office / Office européen des brevets**

(11) **EP 4 306 692 B1**

(12) **EUROPEAN PATENT SPECIFICATION**

(45) Date of publication and mention
of the grant of the patent:
**04.06.2025 Bulletin 2025/23**

(21) Application number: **22184633.0**

(22) Date of filing: **13.07.2022**

(51) International Patent Classification (IPC):
*D01H 13/26* (2006.01)    *B65H 63/06* (2006.01)
*D06H 3/10* (2006.01)    *G01J 3/46* (2006.01)
*G01N 33/36* (2006.01)

(52) Cooperative Patent Classification (CPC):
**D06H 3/10; B65H 63/065; D01H 13/26;**
**G01N 33/365;** B65H 2701/31; G01J 3/50

(54) **ASSESSING THE MANUFACTURING OF TEXTILE BODY USING COLOR PARAMETERS**

BEWERTUNG DER HERSTELLUNG EINES TEXTILKÖRPERS UNTER VERWENDUNG VON FARBPARAMETERN

ÉVALUATION DE LA FABRICATION DE CORPS TEXTILE À L'AIDE DE PARAMÈTRES DE COULEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.01.2024 Bulletin 2024/03**

(73) Proprietor: **Gebrüder Loepfe AG**
**8623 Wetzikon (CH)**

(72) Inventors:
• **Mennicke, Ralph**
**8610 Uster (CH)**
• **Frick, Manuel Bruno**
**8610 Uster (CH)**
• **Bace, Martin**
**8633 Wolfhausen (CH)**
• **Hilzinger, Roger**
**8546 Islikon (CH)**

(74) Representative: **E. Blum & Co. AG**
**Franklinturm**
**Hofwiesenstrasse 349**
**8050 Zürich (CH)**

(56) References cited:
EP-A1- 1 229 323        CN-U- 201 695 253
JP-A- H0 427 851        JP-A- H03 234 861
US-A1- 2007 013 904

## Description

Technical Field

The invention relates to a method for assessing the quality of an elongate textile body, in particular of a yarn, as well as to an apparatus to implement such a method.

Background Art

Typically, color components and other body parameters of a yarn or other type of elongate textile bodies, such as yarn-precursors, are measured by means of a sensor head. In order to assess yarn quality, the parameters are monitored to lie inside predetermined ranges. If events are detected where this condition is not met, the sample is determined to be defective.

Defective sections of yarn may e.g. be removed during the rewinding process in a yarn clearer. While such yarn clearing improves overall yarn quality, it leads to loss of time and material.

JPH03234861A describes a technique to measure the color of a sheet-like textile body at various places and to compare it to a standard color.

US20070/013904A1 describes an apparatus for characterizing the color of a target with light sources of different wavelengths.

JPH0427851A describes a method and apparatus to detect a defect in a fiber structure or film-like article using a color sensor.

Disclosure of the Invention

Hence, it is a general object of the invention to provide a method and apparatus of the type above that allow a better assessment of the quality of the textile body.

This object is achieved by the method and apparatus of the independent claims.

Accordingly, the invention relates to a method for assessing a quality of an elongate textile body, in particular a yarn, that comprises at least the following steps:

- Running the elongate textile body past at least one sensor head: The textile body may e.g. be a yarn precursor, a yarn, or a fabric manufactured from yarn.
- Determining, by means of the sensor head, a set of samples $B_m = (B_{1m} \dots B_{Km})$ of body parameters of the textile body. $K > 1$ is the number of different body parameters, such as the number of different color components. The samples are determined repetitively by means of online measurements, i.e. measurements at one or more locations along the manufacturing chain of e.g. the yarn being manufactured. The body parameters include a color parameter descriptive of at least one color component of the textile body. Advantageously, the color parameter is descriptive of at least two color components in a color space and/or of at least the hue of the color. For example, the body parameters may include the RGB components of a body color measured by the sensor head. $m$ is an index indicative of a time and/or body location a given $B_m$ is associated with, with $m = 1 \dots M$ with $M > 1$, advantageously $M > 100$. The samples may be equal to the actually measured values, but typically they are functions thereof. For example, they may be corrected for range, linearity, and/or offset.
- Using the samples $B_m$ for assessing the quality of a section of the elongate textile body: This step includes at least one of the following steps A and B:

    A) Assessing the "variability" of the color parameter in said section. If the variability is smaller than a desired first variability level, an alert is issued and/or at least part of said section is removed. In this context, "variability" is to be understood as a monotonously increasing function of the variance, of the standard deviation, or of a percentile range of the at least one color component.

    B) Comparing the color parameter in said section against a secondary color. The secondary color may be one of the following:

- If the elongate textile body is to be dyed, it can be a dye color indicative of the color of a dye: In other words, a fault score is calculated as a function of the color parameter and the intended color for dyeing the elongate textile body in a later step.
- If the elongate body is a fancy yarn having a primary color and at least one secondary color, the claimed "secondary color" can correspond to the secondary color of the fancy yarn.

In this context, a "fancy yarn" is a yarn having a primary yarn color and intentional defects having at least one "secondary" yarn color.

As described below, both these measures A and B deviate from the conventional scheme of simply comparing the color parameter against predefined maximum ranges, thereby achieving a more differentiated quality assessment in view of future applications of the textile body.

In particular, step A does, in a sense, the opposite of conventional methods. In conventional methods, a defect is flagged if the variability is large. Rather, step A requires a certain minimum variability to be present in the textile body. This is particularly useful if the yarn resulting from a manufacturing process is intended to be a "mélange yarn", which is a yarn intentionally produced by several differently colored fibers, which gives rise to varying colors along the length of the yarn, or a "fancy yarn". By checking for a minimum variability to be present in the yarn, the quality of such a mélange yarn can be assessed. If the variability becomes too small, a defect may be flagged and/or at least part of the defective section may be removed automatically, e.g. in a yarn clearer. This allows monitoring or automatically clearing mélange-type yarns, something that is not possible by means of conventional yarn monitoring techniques.

As to step B, it allows weighing color deviations differently depending on their hue, e.g. based on the intended dyeing of a yarn. For example, if a yarn is to be dyed in blue, blueish defects may be less of an issue than defects that have a different hue, e.g. red or green defects. Similarly, intended color deviations in fancy yarn can be ignored. Hence, advantageously, the method comprises at least the following steps:

-    Entering, into a control unit of the apparatus, the secondary color: For example, the secondary color may be entered into the control unit manually or by means of a digital data interface to other computing units or software units. The digital data interface e.g. allows to automatically specify the secondary color.
-    Comparing the color parameter in said section against the entered secondary color: The control unit can then automatically compare the color parameter against the secondary color, i.e. it can calculate the fault score as a function of the measured color parameter and the secondary color.

In one embodiment, the comparison of the color parameter against the secondary color may comprise the following steps:

-    Determining the deviation between the color parameter and the "typical color" of the textile body: In this context, the "typical color" may e.g. be the average or median color of the textile body. By calculating the deviation between an actually measured color parameter and the typical color, the "direction" of color deviation in the used color space can be calculated, such as "the yarn should be yellow-white, but this measurement indicates that we have a subsection that deviates towards the blue".
-    Comparing the deviation against the difference between the secondary color and the typical color.

A direct comparison of a measured color parameter and the secondary color would lead to less meaningful results than an indirect comparison by first calculating the deviation and then comparing the deviation to said difference. In the example above, and assuming that the diameter is constant, white fibers seem to have been replaced blue ones. If the yarn is to be dyed blue anyway, such blue fibers matter less than e.g. red fibers.

The present invention also relates to a method for manufacturing a yarn that comprises the method for assessing the quality of an elongate textile body as described herein. The elongate textile body may e.g. be the yarn itself or a yarn precursor.

The method for manufacturing the yarn may in particular comprise the steps of

-    Comparing the color parameter in said section against a dye color indicative of the color of a dye the elongate textile body is to be dyed with: This comparison is used for assessing the quality of the textile body taking into account the later dyeing thereof as described above.
-    Dyeing the yarn with the dye having the dye color.

This combination allows taking the dyeing into account when assessing the quality of the yarn or yarn precursor.

The method for manufacturing the yarn may also comprise the step of removing a section of yarn as a function of the assessed quality of said section. In other words, the assessed quality may be used in a yarn clearer to test if a given section of yarn has to be removed.

As mentioned, the invention also relates to a yarn manufacturing apparatus. This apparatus comprises a plurality of sensor heads and a control unit adapted and structured to carry out the methods of the present invention.

Brief Description of the Drawings

The invention will be better understood and objects other than those set forth above will become apparent when consideration is given to the following detailed description thereof. This description refers to the annexed drawings, wherein:

Fig. 1 is a schematic view of an apparatus for manufacturing yarn,
Fig. 2 shows an example of the normalized sensitivities of the color components measured by a sensor head,
Fig. 3 is schematic diagram of an embodiment of a sensor head,
Fig. 4 shows the distribution of a measured value (A) and the body parameter (B) derived therefrom,
Fig. 5 is a graph depicting the variance of the color as a function of yarn position,
Fig. 6 illustrates criteria for yarn clearing, and
Fig. 7 shows an example of the values of a color parameter as measured for textile body.

Modes for Carrying Out the Invention

*Definitions*

*A body parameter* comprises at least two parameters derived from measurements on the elongate body. It may for example include components from a color parameter (see below) and/or non-optical parameters as mentioned herein.

*A color parameter* comprises at least one, in particular several color components of a color space. Advantageously, it includes at least three color components of a color space. The color space is used to describe the color detected by the sensor head. In this context, the color space advantageously spans at least the visible spectrum, e.g. between wavelengths of 380 and 750 nm. It may, however, also extend into the ultraviolet, e.g. at least as low as 250 nm, and/or into the near infrared, e.g. at least as high as 1.8 $\mu$m. Advantageously, the color parameter is descriptive of at least the hue of the color of the textile body, e.g. in HSV color space.

For example, the components may describe the optical reflection or transmission of the textile body in at least two different spectral ranges. Advantageously, there may be at least three color components indicative of the optical reflection or transmission of the textile body in at least three different spectral ranges of the spectrum between, in particular with each of the at least three different colors falling at least in part into a spectral range of 250 nm and 1.8 $\mu$m, in particular at least in part into the visible spectral range of 380 and 750 nm.

The color space may also be the RGB color space or another color space indicative of the color in three or more different spectral ranges, or it may be the CMY or HSV color space, with the components being at least one or two of the coordinates in these color spaces, e.g. the H coordinate.

*A textile body* may be a yarn precursor, in particular a stream of flocks in the blow room, or a sliver or roving as obtained by carding and drawing, or it may be a yarn as obtained by the spinning process or a fabric manufactured from such yarn. In some embodiments, it may also be a product manufactured from the yarn. Advantageously, though, at least the textile body sampled at the claimed sensor head is a yarn.

The *yarn manufacturing apparatus* may at least comprise one of a blow room, a carding machine, a drawing machine, a spinning machine, a winder, and a yarn clearer. Advantageously, the apparatus comprises at least a yarn clearer.

*Overview*

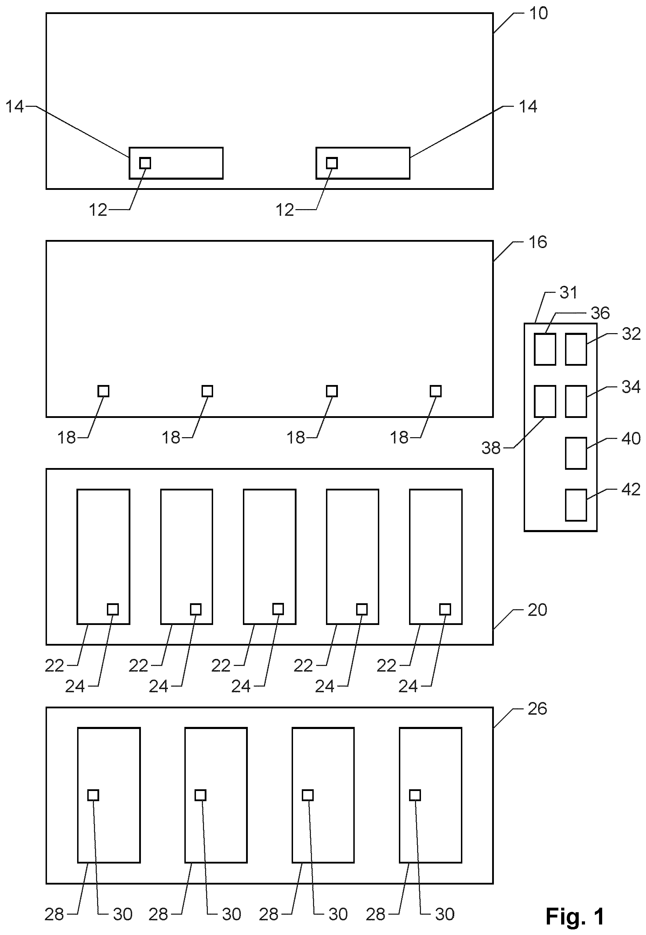

Fig. 1 schematically shows some elements of a cotton spinning mill as an example of a yarn manufacturing apparatus.

As known to the skilled person, such a spinning mill comprises a blow room 10, where bales of cotton are opened into flocks, which may then be precleaned as well as fine cleaned. In this process, one or more streams of flocks are generated.

The stream(s) of flocks may be run past sensor heads 12, e.g. in the fine cleaners 14 of the blow room 10. The function of these sensor heads 12 will be described below.

The mill further comprises a carding and drawing section 16, where the fibers in the flocks are separated, straightened, and formed into slivers and rovings. The slivers or rovings may again be run past sensor heads 18.

In a next step, the products from carding and rowing section 16 are fed to a spinning section 20, where they are spun into yarns in a plurality of spinning units 22. The yarns may again be run past sensor heads 24.

Finally, the yarns, e.g. wound on cops, are fed to a winding section 26 having a plurality of yarn clearers 28. In each yarn clearer 28, the yarn is run past a sensor head 30, e.g. while being wound from cops onto a bobbin.

The apparatus further comprises a control unit 31 controlling the operation of the individual components. In Fig. 1, it is depicted as a single element, but it may also be distributed and/or have sub-sections attributed to the different parts of the apparatus.

Typically, control unit 31 comprises at least one CPU 32, memory 34, input interfaces 36 for receiving sensor signals, e.g. from the various sensor heads as well as from other sensors of the apparatus, output interfaces 38 for controlling actuators of the apparatus, at least one input control 40 for receiving user input, and at least one display 42 for displaying information to the operator.

Control unit 31 may perform various functions using program code and parameters stored in memory 34. In particular, it is programmed to carry out the methods as described herein.

*Sensor heads*

As mentioned above, the apparatus comprises at least one or a plurality of sensor heads 12, 18, 24, 30. Each of these sensor heads is adapted to measure samples $B_{km}$ of one or more of the body parameters of the textile body, where index k = 1 ... K indicates the parameter and index m is indicative of the time and/or yarn position to which the samples are attributed.

The number K of body parameters is advantageously larger than 1. A single sensor head may be adapted to measure all K body parameters, or different sensor heads may be provided to measure different subsets of the body parameters.

The samples of the body parameters can be written as a vector $B_m =(B_{1m} ... B_{Km})$. They are determined repetitively and online, i.e. they are determined on the textile body while it is being processed by the apparatus in a manufacturing process.

Advantageously, the body parameters include the color components $C_1 ... C_N$ of a color parameter $C = (C_1 ... C_N)$ of the textile body, with N being at least 2, in particular at least 3. Each such color component $C_n$ may describe (i.e. may depend on) the interaction of the textile body with light of a given spectral range $W_n$, wherein the spectral ranges of the different color components differ from each other.

In one embodiment, the color space may be based on different spectral components in a spectral range as specified above.

Advantageously, each color component $C_n$ describes the optical reflectivity of the textile body in the given spectral range. However, in another embodiment, each color component $C_n$ may describe the optical transmission of the textile body at the given spectral range.

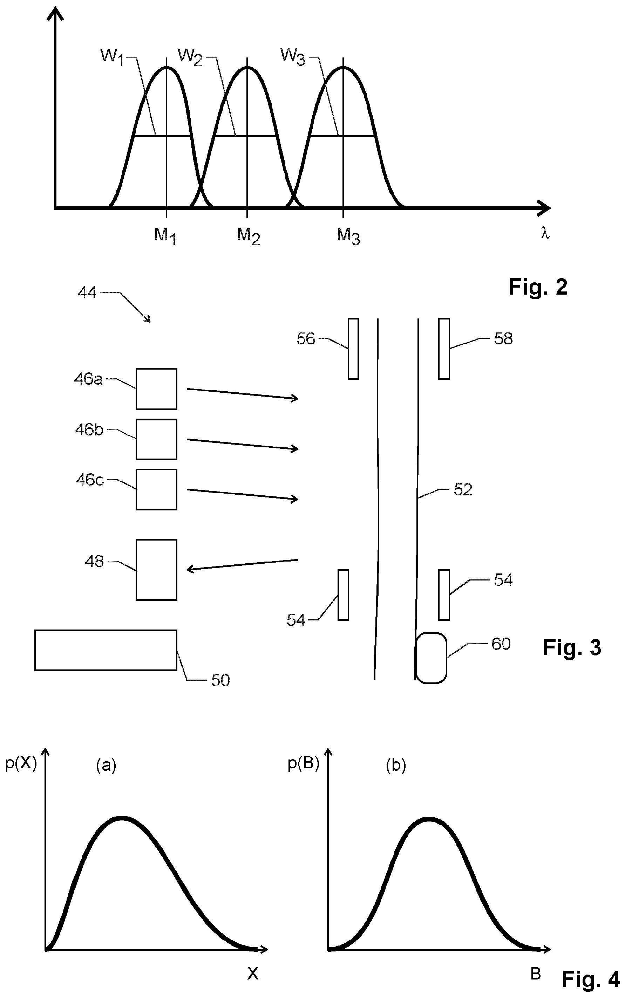

The spectral ranges are advantageously non-overlapping and/or span a substantial part of the visible spectrum. This is illustrated in Fig. 2, which shows the normalized sensitivities of the color components $C_n$ with n = 1 ... N (in this case for N = 3) as a function of the wavelength λ. For each color component, $W_n$ denotes the spectral range or width at half maximum and $M_n$ the wavelength of maximum sensitivity.

In order to be non-overlapping, there should advantageously be at least two, in particular at least three, color components whose spectral ranges $W_n$ have a mutual overlap by less than 0.25, with the mutual overlap $O_{ij}$ of two spectral ranges $W_i$ and $W_j$ being defined by

$$O_{ij} = 2* W_{ij} / (W_i + W_j),$$

with $W_{ij}$ being the range where $W_i$ and $W_j$ overlap.

Advantageously, at least one spectral range, in particular at least two of them, should have a width $W_n$ smaller than 50 nm for good color selectivity.

In order to span a substantial part of the visible spectrum, there should advantageously be a least two, in particular at least three, color components whose maximum sensitivities $M_n$ are at least 50 nm away from each other.

Advantageously, at least one maximum sensitivity $M_n$ is in the violet, blue, or green spectral range, i.e. below 550 nm, and at least one maximum sensitivity $M_n$ is in the red spectral range, i.e. above 600 nm. In particular, there is advantageously one maximum sensitivity in the blue or violet spectral range below 500 nm, at least one maximum sensitivity in the green or yellow/orange spectral range between 500 nm and 600 nm, and at least one maximum sensitivity in the red spectral range above 600 nm.

Fig. 3 shows a schematic diagram of a possible embodiment of one sensor head 44, which, in the present embodiment, is adapted to measure color components as well as non-optical parameters of the textile body.

In the shown embodiment, it comprises three light sources 46a, 46b, 46c and a light detector 48. The light sources 46a, 46b, 46c emit light at different spectral ranges, corresponding e.g. to those in Fig. 2. Light sensor 48 is sensitive at all these spectral ranges. Control circuitry 50 is provided for operating the light sources 46a, 46b, 46c to e.g. emit light pulses sequentially and for detecting the response at light sensor 48 for each such light pulse.

The light from the light sources 46a, 46b, 46c falls on the textile body 52 to be inspected, interacts with the same, and, after this interaction, it is detected by light sensor 48. The interaction is advantageously a reflection, i.e. light sensor 48 detects light from the light sources 46a, 46b, 46c that has been reflected from textile body 52.

By attributing the measured light pulses to the individual light sources 46a, 46b, 46c, control circuitry 50 is able to measure the color components $C_n$ for the respective spectral ranges $W_n$.

In yet another embodiment (not shown), three separate light detectors sensitive only in the different spectral ranges $W_n$ may be used, e.g. in combination with a broadband light source.

An example of a suitable sensor head for color components is e.g. described in EP3748343A1.

The sensor head of Fig. 3 may further comprise a capacitive sensor 54, which is adapted to measure the electrical capacitance of a measurement volume comprising textile body 52. Such a sensor is e.g. described in EP3751282A1. This sensor is used to determine a capacitance of the textile body.

The sensor head of Fig. 3 may further comprise a thickness sensor using a light source 56 and a light detector 58 to estimate the diameter of the textile body using shadowing techniques as e.g. described in EP3748342A1.

The sensor head of Fig. 3 may further comprise a triboelectric sensor 60 for measuring a triboelectric property of the textile body, such as e.g. described in CH532526 or US6650959.

*Operation*

In operation, textile body 52 is run past sensor head 44, and sensor head 44 generates measured values $X_{km}$ at times or body locations indexed by index m. The measured values $X_{km}$ may have been subject to preprocessing, e.g. using filtering, downsampling, inverting, offsetting and/or averaging techniques, etc.

A set of samples $\mathbf{B}_m = (B_{1m} \dots B_{Km})$ is determined from the measured values, e.g. using

$$B_{km} = F_k(X_{km}). \tag{1}$$

The functions $F_k$ may be the identity functions, i.e. $B_{km} = X_{km}$, but advantageously, the functions $F_k$ may be selected such that they fulfill at least one of the following conditions:

A) They offset and/or scale the measured values $X_{km}$ to map them into equal numeric ranges for a numerically more stable processing in the following steps.

B) They offset the measured values $X_{km}$ to map them into ranges symmetrical to zero, again to make the following steps easier to implement.

C) The functions $F_k$ may be non-linear, i.e. the samples $B_{km}$ of the body parameters are non-linear transforms of the measurements $X_{km}$. **In** particular, this allows making the statistical distribution of the first set body parameters $B_k$ symmetric, and advantageously even Gaussian, in respect to a center point, e.g. zero. This is illustrated in Fig. 4, where graph (a) depicts an asymmetric probability distribution of a given measurement X and graph (b) depicts the nonlinear transform of X into the body parameter B that has symmetric probability distribution.

Condition C) may also make the distribution along the principal components (see below) symmetric.

Condition C) may e.g. be implemented by:

- Step 1) Writing

$$F_k(X_{km}) = X_{km}{}^a, \tag{2}$$

wherein a is an exponential to be determined by the following steps.

- Step 2a) Calculating, for a plurality of the measurement values $X_{km}$, the values of $F_k(X_{km})$, and then calculating a statistical measure of the asymmetry of the distribution of $F_k(X_{km})$, in particular the skewness, and finding the value of the exponent a where the asymmetry is smallest, or

- Step 2b) Calculating, for a plurality of the measurement values $X_{km}$, the values of $F_k(X_{km})$, performing a statistical normality test on the $F_k(X_{km})$, and finding the value of the exponent a where the distribution of the $F_k(X_{km})$ is closest to a normal distribution.

Steps 2a) and/or 2b) may also be applied to other types of functions $F_k(X_{km})$, in addition to or alternatively to Eq. (2), e.g.

$$F_k(X_{km}) = \log(X_{km}). \tag{2'}$$

In another embodiment, for a set $X_{km}$ with m = 1 to M >> 1, several different quantiles may be calculated and the scale (X-axis) may be mapped nonlinearly to turn the histogram of the set into a normal distribution.

In more general terms, therefore, the present method may comprise the steps of

- measuring, by means of the sensor head, a plurality of measured values $X_{km}$ and
- calculating, from the measured values $X_{km}$, the samples $\mathbf{B}_m$ by means of non-linear transforms $F_k$, wherein the non-linear transforms $F_k$ are adapted to make the distribution of the samples $\mathbf{B}_m$ symmetric, in particular to make said distribution normal.

It must be noted that Eq. (1) assumes that each measured value $X_{km}$ is mapped into a corresponding sample $B_{km}$ of the body parameter. However, preprocessing the measured values may e.g. also comprise the step of combining two or more different measured values $X_{k'm}$, $X_{k''m}$ into a single sample $B_{km}$, i.e.

$$B_{km} = F_k(X_{k'm}, X_{k''m}). \qquad (1')$$

The set of samples determined in this manner can then be used for a plurality of applications described in the following sections.

*Mélange yarns*

As mentioned above, mélange yarns are yarns produced by intentionally combining several differently colored fibers, which gives rise to different, varying colors along the length of the yarn.

Mélange yarns may be blended mélange yarns consisting of different fiber materials or they may be non-blended mélange yarns consisting of the same but differently colored fiber materials. In the context of the present description and claims, the term "mélange yarn" is advantageously understood to incorporate "fancy yarns", in particular "slub yarns".

Such mélange yarns typically have body parameters, in particular color parameters, that should fluctuate along the length of the body.

In order to assess the quality of a yarn, it is therefore proposed to assess the variability of the color parameter in a section of the textile body, in particular of the finished yarn, and e.g. issuing an alert and/or removing at least part of said section if the variability is smaller than a desired first variability level.

As mentioned above, "variability" is to be understood as a value that is descriptive of how strongly the color parameter varies, with the variability becoming larger as the variation of the color parameter increases. According to the claims, the variability is a monotonously increasing, in particular a strictly monotonously increasing, function of the variance, of the standard deviation, or of a percentile range of one or more of the color components.

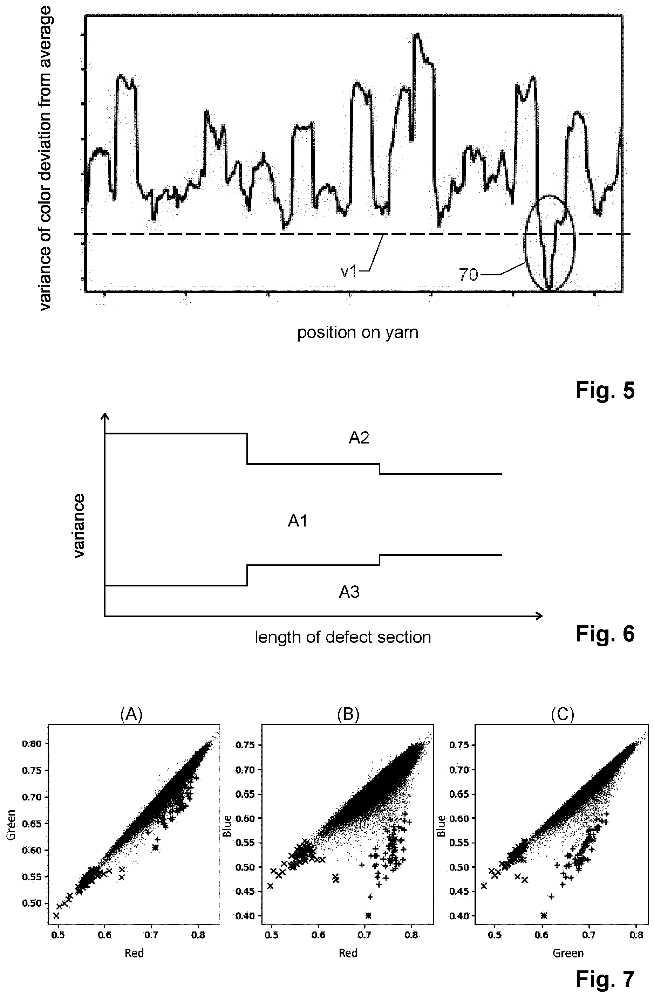

This is illustrated in Fig. 5, which shows a graph with a vertical axis corresponding to the statistical variance of the deviation between the color and the average color as determined over a length of e.g. 10 cm of yarn. The horizontal axis is the position on the yarn.

In this example, the variability between the color and the average color is calculated as the variance over a 10 cm window for a given color component.

As can be seen, the calculated variance is typically above a minimum variance v1. However, at a region 70, it is found to drop below the minimum variance v1.

Therefore, for example, the difference between the variance as calculated and the minimum variance v1 can be used as a quality measure for a mélange yarn. As long as the variance is larger than the minimum variance v1, quality is sufficient. If, however, the variance falls below the minimum variance v1, the corresponding section of textile body may be flagged as potentially defective and/or be removed.

In another example, there may not be a single minimum variance v1, but several minimum variances may be used depending on the length of the fault. In other words, a short section of yarn having a low variance may not be flagged as a fault while a long section of yarn having the same low variance is flagged as a fault.

Similarly, an upper limit of the variance may be provided as well in order to flag sections of high variability as defective. Again, this upper limit may depend on the length of the given section.

Fig. 6 is an illustration how different minimum and (optionally) maximum values of the variance can be specified depending on the length of a fault. Regions A2 and A3 are the areas where a fault is flagged or removed while region A1 is the area of permissible variations. For short potential defects, the limits of the variance may be set further apart than for long defects.

Advantageously, the variance is calculated over a section of textile body, in particular yarn, that has a length between 10 cm and 500 m, in particular between 20 cm and 250 m.

Hence, advantageously, the variability is derived over a yarn length between 10 cm and 500 m, in particular between 20 cm and 250 m..

In another embodiment, the variability can be obtained from a "spectral analysis" of the color parameter (or at least one of its color components) as a function of time or body position. In particular, the method may comprise the following steps:

- Recording the color parameter as a function of time or position along the textile body.
- Determining at least one spectral value indicative of a frequency distribution of the color parameter. In this context, "frequency" designates the frequency in Hertz (e.g. in units of 1/s) of the color parameter or the spatial frequency (e.g. in units of 1/m) of the color parameter. The spectral value may e.g. be the integral, median, or maximum value of the spectrum of one or more of the color components over a given frequency range. This spectrum can e.g. be expressed as the power spectral density over the given frequency range, see https://en.wikipe-dia.org/wiki/Spectral_density.
- Determining the variability using the spectral value. In other words, the variability is determined as a mathematical function of this spectral value (and, optionally, of further parameters). For example, the variability can be equal to the spectral value or be calculated as a monotonous function thereof.

As mentioned above, an alert is advantageously issued if the variability is smaller than the desired first variability level. In addition to this, an alert may also be issued if the variability is larger than a second variability level. This may also indicate that the yarn may be faulty because its color varies too much.

In yet another embodiment, the variability of the color parameter may be calculated along a given direction in color space only, e.g. by projecting each value of the color parameter Cp onto a line extending along that direction in order to calculate a projection value p, e.g. by calculating the scalar product of

$$p = \mathbf{Cp} \cdot \mathbf{e},$$

wherein e is a unit vector along the given direction in color space. The variability may then be calculated as the variability of the projection value p. Again, the variability may e.g. be the monotonously increasing function of the variance, standard deviation, or percentile range of the projection value p.

The given direction in color space may e.g. be a predefined color variance direction of the yarn. Advantageously, though, the given direction is one of the principal components $\mathbf{w}_n$ of a dataset comprising a plurality of the measured color parameters $\mathbf{C}p$ of the textile body. Advantageously, the given direction is the principal component $\mathbf{w}_n$ along which the highest variance is observed. Methods for calculating the principal component(s) are discussed in the section "principal component analysis" below.

*Dye color or secondary color*

As mentioned above, the assessment of yarn quality may also take into account a secondary color, in particular a color indicative of how a yarn (or other textile product) is to be dyed after its manufacturing process.

For example, and as mentioned, if a cotton yarn is to be dyed blue, bluish foreign fibers may be tolerated to a larger degree than red ones.

Hence, advantageously, the color parameter in a section of textile body being examined is compared against a secondary color Cd, the dye color indicative of the color that will be used for dyeing. In this context, "compared against" is to be understood as calculating a fault score that is a function of the color parameter and the intended color for dyeing.

In one embodiment, this includes determining the typical color Ct of the textile body, which may e.g. be the average or median color of the textile body as measured along a given length of the textile body.

In a next step, the deviation ΔC between the color parameter Cp as determined from the section being investigated and the typical color is calculated, i.e.

$$\Delta\mathbf{C} = \mathbf{Cp} - \mathbf{Ct}. \tag{3}$$

(ΔC, Ct, and Cp are vectors in a color space, such as RGB.)

Now, the deviation ΔC is compared against the difference Cd - Ct between the dye color Cd and the typical color Ct. This can e.g. be done by calculating the components ΔC1 and ΔC2 of the deviation ΔC along Cd - Ct and perpendicularly thereto

$$\Delta C1 = \Delta\mathbf{C} \cdot (\mathbf{Cd} - \mathbf{Ct}) / \| \mathbf{Cd} - \mathbf{Ct} \| \tag{4}$$
$$\Delta C2 = (\|\Delta\mathbf{C}\|^2 - \Delta C1^2)^{1/2}$$

with the dot · denoting the scalar product of two vectors and with the vertical lines $\|...\|$ denoting an appropriate norm, e.g. the Euclidean norm, that is the length of a vector.

Then, a fault score $Q(\Delta C1, \Delta C2)$ is calculated as a function of the components $\Delta C1$ and $\Delta C2$, with the fault score being indicative of the likelihood that a given section of textile body is a defect. The fault score depends less on $\Delta C1$ than on $\Delta C2$, i.e.

$$|\partial Q / \partial \Delta C1| < |\partial Q / \partial \Delta C2| \qquad (5)$$

Inequality (5) should be maintained at least for values of $\Delta C1 / \|\Delta C\| < 1$, in particular $0.9 < \Delta C1 / \|\Delta C\| < 1$, i.e. as long the offset of the color parameter towards the dye color is not too large.

In one advantageous embodiment,

$$Q(\Delta C1, \Delta C2) = \begin{cases} \Delta C2 & for\ 0 < \Delta C1 < \|\mathbf{C}d - \mathbf{C}t\| \\ ((\Delta C1 - \|\mathbf{C}d - \mathbf{C}t\|)^2 + \Delta C2^2)^{1/2} & for\ \|\mathbf{C}d - \mathbf{C}t\| < \Delta C1 \\ (\Delta C1^2 + \Delta C2^2)^{1/2} & else \end{cases}$$

Hence, in more general terms, the comparison of the deviation $\Delta C$ against the typical color Ct may comprise the following steps:

- Calculating components $\Delta C1$ and $\Delta C2$ of the deviation $\Delta C$ along the difference Cd - Ct between the dye color Cd and the typical color Ct and perpendicularly thereto and
- Calculating a fault score $Q(\Delta C1, \Delta C2)$ from the components $\Delta C1$ and $\Delta C2$ that depends less on $\Delta C1$ than on $\Delta C2$.

Alternatively, instead of using the deviation $\Delta C$ between the color parameter Cp as determined from the section being investigated and the typical color, the fault score may also only depend on the difference between the color parameter Cp of the tested section and the dye color Cd, i.e. Cp - Cd, in particular if the typical color Ct of the textile body is close to white.

Note: As mentioned above, the secondary color may also represent the secondary color if the body is a fancy yarn having a primary color as well as a secondary color. The primary color is indicative of the color along the majority of the yarn, while the secondary color is indicative of intentional color defects in the yarn.

*Principal component analysis*

As mentioned, principal component analysis may be used to assess the yarn.

The concept behind this idea is illustrated with reference to Fig. 7, which shows the components of the color parameter measured for a given textile body during a run. In this case, three color components were measured, one for a blue spectral range, one for a green spectral range, and one for a red spectral range. Each one of the dots, +-crosses, and x-crosses in Fig. 7 stands for the color components measured at one time.

As can be seen, the vast majority of the values (the ones that are shown as dots) are confined to a "main" group denoted by dots, but there are also two distinct "outlier" groups, one of which is denoted by the +-crosses and the other one by the x-crosses.

In the present case, the main group corresponds to color values close to the expected, desired color of the body while the outlier groups indicate specific, distinct defects in the body.

As can be seen, though, the main group does not have symmetric distribution. Rather, it is elongate, stretching along the white-black direction (luminance) of the color space. This is a typical behavior e.g. for cotton, where the material may vary in luminance but less in hue and saturation. For this reason, variations in hue or saturation are usually less acceptable than variations in luminance.

Similar issues may arise for other types of materials, such as wool, silk, or synthetic fibers. Especially mélange yarns, as they are a blend of fibers which can have different colors, often show a variation especially between the two colors of their composing fibers.

Hence, advantageously, the present method includes the determination of the principal components $w_n$ of the set of samples $B_m$, i.e. the determination of the main direction along which the sample values vary.

In the example of Fig. 7, a first principal component extends substantially along the luminance direction (i.e. between RGB = (0,0,0) and RGB = (1,1,1)), while the other two principal components extend perpendicularly thereto.

*Notes*

In the present system, the quality of an elongate textile body 52, such as a yarn precursor or a yarn, is assessed. The textile body is run past a sensor head 20, 18, 24, 30; 44. At a first plurality of times, samples of the body parameters of the textile body 52 are measured. The parameters may include color components.

A minimum variation of the parameters may be requested when assessing the quality of mélange yarns.

The color of a dye to be applied to the manufactured yarn may be taken into account in order to reduce the number of flagged or cleared yarn sections.

While there are shown and described presently preferred embodiments of the invention, it is to be distinctly understood that the invention is not limited thereto but may be otherwise variously embodied and practiced within the scope of the following claims.

**Claims**

1.  A method for assessing a quality of an elongate textile body comprising the steps of

    running the elongate textile body (52) past at least one sensor head (12, 18, 24, 30; 44),
    determining, by means of the sensor head (12, 18, 24, 30; 44), a set of samples $B_m = (B_{1m} ... B_{Km})$ of body parameters of the textile body (52) with K > 1 by repetitively carrying out online measurements, wherein said body parameters include a color parameter (Cp) descriptive of at least one color component, in particular several color components, of the textile body (52), wherein m = 1 ... M with M > 1
    using the samples $B_m$ for assessing the quality of a section of the elongate textile body,
    **characterized in that** the quality of the section is assessed by at least one of

    A) assessing a variability of the color parameter (Cp) in said section and issuing an alert and/or removing at least part of said section if the variability is smaller than a desired first variability level, wherein the variability is a monotonously increasing function of a variance, of a standard deviation, or of a percentile range of the at least one color component, and/or
    B) comparing the color parameter (Cp) in said section against a secondary color (Cd) indicative of the color of a dye the elongate textile body (52) is to be dyed with or of a secondary color if the textile body is a fancy yarn.

2.  The method of claim 1 wherein the color parameter (Cp) is descriptive of at least at least two color components in a color space.

3.  The method of any of the preceding claims comprising the steps of

    assessing the variability of the color parameter (Cp) in said section and
    issuing the alert and/or cutting out at least part of the section if the variability is smaller than the desired first variability level.

4.  The method of any of the preceding claims wherein the variability is derived over a textile body length between 1 cm and 100 cm, in particular between 3 cm and 50 cm.

5.  The method of any of the preceding claims comprising the steps of

    recording the color parameter (Cp) as a function of time and/or position along the textile body,
    determining at least one spectral value indicative of a frequency distribution of the color parameter (Cp), and
    determining the variability using the spectral value.

6.  The method of any of the preceding claims comprising the steps of

    entering, into a control unit (31), the secondary color (Cd),
    comparing, by means of the control unit (31), the color parameter (Cp) in said section against entered secondary color (Cd).

7.  The method of any of the preceding claims comprising the steps of

- determining a deviation ($\Delta C$) between the color parameter (Cp) and a typical color (Ct) of the textile body and
- comparing the deviation ($\Delta C$) against a difference between the secondary color (Cd) and the typical color (Ct).

8. The method of claim 7 wherein comparing the deviation ($\Delta C$) against the typical color (Ct) comprises the steps of

- calculating components $\Delta C1$ and $\Delta C2$ of the deviation ($\Delta C$) along a difference between the secondary color (Cd) and the typical color (Ct) and perpendicularly thereto and
- calculating a fault score $Q(\Delta C1, \Delta C2)$ from the components $\Delta C1$ and $\Delta C2$ that depends less on $\Delta C1$ than on $\Delta C2$.

9. The method of any of the preceding claims comprising the step of issuing an alert and/or removing at least part of said section if the variability is larger than a second variability level.

10. The method of any of the preceding claims comprising the step of calculating the variability along a given direction in color space.

11. The method of claim 10 wherein the given direction is one a principal component ($w_n$) of a dataset comprising a plurality of the measured color parameters ($Cp$) of the body.

12. The method of any of the preceding claims, comprising the step of determining principal components $w_n$ of the set of samples $B_m$.

13. The method of any of the preceding claims comprising the steps of

measuring, by means of the sensor head (12, 18, 24, 30; 44), a plurality of measured values ($X_{km}$) and calculating, from the measured values ($X_{km}$), the samples $B_m$ by means of non-linear transforms ($F_k$), wherein the non-linear transforms ($F_k$) are adapted to make a distribution of the samples $B_m$ symmetric, in particular to make said distribution normal.

14. The method of any of the preceding claims comprising the step of using the assessed quality for automatically displaying an alert to a user.

15. A method for manufacturing a yarn comprising the method of any of the preceding claims.

16. The method of claim 15 comprising the steps of

comparing the color parameter ($Cp$) in said section against a dye color (Cd) indicative of the color of a dye the elongate textile body (52) is to be dyed with, and
dyeing the yarn with the dye having the dye color ($Cd$).

17. The method of any of the preceding claims 15 or 16 comprising the step of removing a section of yarn as a function of the assessed quality of said section.

18. A yarn manufacturing apparatus comprising

at least one sensor head (12, 18, 24, 30; 44) and
a control unit (31) **characterized by** being adapted and structured to assess the quality of the yarn by carrying out the method of any of the preceding claims.

**Patentansprüche**

1. Verfahren zur Bewertung der Qualität eines langgestreckten Textilkörpers, das die folgenden Schritte umfasst

Vorbeiführen des langgestreckten Textilkörpers (52) an mindestens einem Sensorkopf (12, 18, 24, 30; 44),
Ermitteln eines Satzes von Abtastwerten $B_m = (B_{1m} \dots B_{Km})$ von Körperparametern des textilen Körpers (52) mit K > 1 mittels des Sensorkopfes (12, 18, 24, 30; 44) durch wiederholtes Durchführen von Echtzeit-Messungen, wobei die Körperparameter einen Farbparameter ($Cp$) umfassen, der mindestens eine Farbkomponente, insbesondere mehrere Farbkomponenten, des textilen Körpers (52) beschreibt, wobei m = 1 ... M mit M > 1,

Verwendung der Abtastwerte $\mathbf{B}_m$ zur Bewertung der Qualität eines Abschnitts des länglichen Textilkörpers, **dadurch gekennzeichnet, dass** die Qualität des Abschnitts durch mindestens eines der folgenden Kriterien bewertet wird

A) Bewerten einer Variabilität des Farbparameters ($\mathbf{C}$p) im Abschnitt und Ausgeben einer Warnung und/oder Entfernen mindestens eines Teils des Abschnitts, wenn die Variabilität kleiner als ein gewünschtes erstes Variabilitätsniveau ist, wobei die Variabilität eine monoton ansteigende Funktion einer Varianz, einer Standardabweichung oder eines Perzentilbereichs der mindestens einen Farbkomponente ist, und/oder
B) Vergleichen des Farbparameters ($\mathbf{C}$p) in dem genannten Abschnitt mit einer Sekundärfarbe ($\mathbf{C}$d), die die Farbe eines Farbstoffs angibt, mit dem der längliche Textilkörper (52) gefärbt werden soll, oder einer Sekundärfarbe, wenn der Textilkörper ein Effektgarn ist.

2. Verfahren nach Anspruch 1, wobei der Farbparameter ($\mathbf{C}$p) mindestens zwei Farbkomponenten in einem Farbraum beschreibt.

3. Das Verfahren nach einem der vorhergehenden Ansprüche umfasst die Schritte

die Bewertung der Variabilität des Farbparameters ($\mathbf{C}$p) im Abschnitt und
Ausgabe der Warnmeldung und/oder Herausschneiden zumindest eines Teils des Abschnitts, wenn die Variabilität kleiner ist als das gewünschte erste Variabilitätsniveau.

4. Verfahren nach einem der vorhergehenden Ansprüche, wobei die Variabilität über eine Textilkörperlänge zwischen 1 cm und 100 cm, insbesondere zwischen 3 cm und 50 cm abgeleitet wird.

5. Verfahren nach einem der vorhergehenden Ansprüche umfassend die Schritte

Aufzeichnung des Farbparameters (Cp) als Funktion der Zeit und/oder der Position entlang des Textilkörpers,
Bestimmung mindestens eines Spektralwertes, der eine Frequenzverteilung des Farbparameters (Cp) angibt, und
Bestimmung der Variabilität anhand des Spektralwertes.

6. Verfahren nach einem der vorhergehenden Ansprüche umfassend die Schritte

Eingabe der Sekundärfarbe ($\mathbf{C}$d) in eine Steuereinheit (31),
Vergleich, mittels der Steuereinheit (31), des Farbparameters ($\mathbf{C}$p) im genannten Abschnitt mit der eingegebenen Sekundärfarbe ($\mathbf{C}$d).

7. Verfahren nach einem der vorhergehenden Ansprüche umfassend die Schritte

- Bestimmung einer Abweichung ($\Delta\mathbf{C}$) zwischen dem Farbparameter (Cp) und einer typischen Farbe ($\mathbf{C}$t) des Textilkörpers und
- Vergleich der Abweichung ($\Delta\mathbf{C}$) mit einer Differenz zwischen der Sekundärfarbe ($\mathbf{C}$d) und der typischen Farbe ($\mathbf{C}$t).

8. Verfahren nach Anspruch 7, wobei der Vergleich der Abweichung ($\Delta\mathbf{C}$) mit der typischen Farbe ($\mathbf{C}$t) die folgenden Schritte umfasst

- Berechnung von Komponenten $\Delta C1$ und $\Delta C2$ der Abweichung ($\Delta\mathbf{C}$) entlang einer Differenz zwischen der Sekundärfarbe ($\mathbf{C}$d) und der typischen Farbe ($\mathbf{C}$t) und senkrecht dazu und
- Berechnung eines Fehler-Scores $Q(\Delta C1, \Delta C2)$ aus den Komponenten $\Delta C1$ und $\Delta C2$, der weniger von $\Delta C1$ als von $\Delta C2$ abhängt.

9. Das Verfahren nach einem der vorhergehenden Ansprüche umfassend den Schritt, eine Warnung auszugeben und/oder zumindest einen Teil des Abschnitts herauszuschneiden, wenn die Variabilität grösser als ein zweites Variabilitätsniveau ist.

10. Verfahren nach einem der vorhergehenden Ansprüche umfassend den Schritt der Berechnung der Variabilität entlang einer bestimmten Richtung im Farbraum.

**11.** Verfahren nach Anspruch 10, wobei die gegebene Richtung eine Hauptkomponente ($w_n$) eines Datensatzes ist, der eine Vielzahl der gemessenen Farbparametern (**C**p) des Körpers umfasst.

**12.** Verfahren nach einem der vorhergehenden Ansprüche, umfassend den Schritt der Bestimmung der Hauptkomponenten $w_n$ der Abtastwerte $\mathbf{B}_m$.

**13.** Das Verfahren nach einem der vorhergehenden Ansprüche umfassend die Schritte

Messen einer Vielzahl von Messwerten ($X_{km}$) mit Hilfe des Sensorkopfes (12, 18, 24, 30; 44) und
Berechnen, ausgehend von den Messwerten ($X_{km}$), der Abtastwerte $\mathbf{B}_m$ mittels nichtlinearer Transformationen ($F_k$), wobei die nichtlinearen Transformationen ($F_k$) dazu ausgestaltet sind, eine Verteilung der Abtastwerte $\mathbf{B}_m$ symmetrisch zu machen, insbesondere diese Verteilung normal zu machen.

**14.** Verfahren nach einem der vorhergehenden Ansprüche umfassend den Schritt der Verwendung der bewerteten Qualität zur automatischen Anzeige einer Warnung an einen Benutzer.

**15.** Verfahren zur Herstellung eines Garns, umfassend das Verfahren nach einem der vorhergehenden Ansprüche.

**16.** Verfahren nach Anspruch 15 umfassend die Schritte

Vergleichen des Farbparameters (Cp) im Abschnitt mit einer Farbstofffarbe (Cd), die die Farbe eines Farbstoffs angibt, mit dem der längliche Textilkörper (52) gefärbt werden soll, und
Färben des Garns mit dem Farbstoff mit der Farbstofffarbe (**C**d).

**17.** Verfahren nach einem der vorhergehenden Ansprüche 15 oder 16 umfassend den Schritt des Entfernens eines Garnabschnitts in Abhängigkeit von der bewerteten Qualität des Abschnitts.

**18.** Vorrichtung zur Herstellung von Garn mit

mindestens einem Sensorkopf (12, 18, 24, 30; 44) und
eine Steuereinheit (31), die **dadurch gekennzeichnet ist, dass** sie dazu ausgestaltet und strukturiert ist, die Qualität des Garns zu bewerten, indem sie das Verfahren nach einem der vorhergehenden Ansprüche durchführt.

**Revendications**

**1.** Un procédé pour évaluer une qualité d'un corps textile allongé, comprenant les étapes suivantes

passer le corps textile allongé (52) devant au moins une tête de capteur (12, 18, 24, 30, 44),
déterminer un ensemble de valeurs d'échantillonnage $\mathbf{B}_m = (B_{1m} ... B_{Km})$ de paramètres de corps du corps textile (52) avec K > 1 à l'aide de la tête de capteur (12, 18, 24, 30, 44) par réalisation répétée de mesures en temps réel, les paramètres de corps comprenant un paramètre de couleur (**C**p) qui décrit au moins une composante de couleur, en particulier plusieurs composantes de couleur, du corps textile (52), m = 1 ... M avec M > 1,
utiliser les valeurs d'échantillonnage $\mathbf{B}_m$ pour évaluer la qualité d'une section du corps textile allongé, **caractérisé en ce que** la qualité de la section est évaluée à l'aide d'au moins l'un des critères suivants

A) évaluer une variabilité du paramètre de couleur (**C**p) dans ladite section et emmètre un avertissement et/ou un enlèvement d'au moins une partie de la section lorsque la variabilité est inférieure à un premier niveau de variabilité souhaité, la variabilité étant une fonction monotone croissante d'une variance, d'un écart type ou d'une plage de percentiles de la au moins une composante de couleur, et/ou
B) comparer le paramètre de couleur (**C**p) dans ladite section avec une couleur secondaire (**C**d) qui indique la couleur d'un colorant avec lequel le corps textile allongé (52) doit être teint, ou une couleur secondaire si le corps textile est un fil à effet.

**2.** Le procédé selon la revendication 1, dans lequel le paramètre de couleur (**C**p) décrit au moins deux composantes de couleur dans un espace colorimétrique.

**3.** Le procédé selon l'une des revendications précédentes, comprenant les étapes

d'évaluer la variabilité du paramètre de couleur (Cp) dans la section et
d'emmètre l'avertissement et/ou la découpe d'au moins une partie de la section lorsque la variabilité est inférieure au premier niveau de variabilité souhaité.

**4.** Le procédé selon l'une des revendications précédentes, la variabilité étant dérivée sur une longueur de pièce textile comprise entre 1 cm et 100 cm, en particulier entre 3 cm et 50 cm.

**5.** Le procédé selon l'une des revendications précédentes, comprenant les étapes suivantes

enregistrer le paramètre de couleur ($\mathbf{C}$p) en fonction du temps et/ou de la position le long du corps textile, déterminer au moins une valeur spectrale qui indique une distribution de fréquences du paramètre de couleur ($\mathbf{C}$p), et
déterminer la variabilité à l'aide de la valeur spectrale.

**6.** Le procédé selon l'une des revendications précédentes, comprenant les étapes suivantes

entrer la couleur secondaire ($\mathbf{C}$d) dans une unité de commande (31),
comparer, à l'aide de l'unité de commande (31), le paramètre de couleur (Cp) dans ladite section avec la couleur secondaire entrée (Cd).

**7.** Le procédé selon l'une des revendications précédentes, comprenant les étapes suivantes

- déterminer un écart ($\Delta\mathbf{C}$) entre le paramètre de couleur ($\mathbf{C}$p) et une couleur typique ($\mathbf{C}$t) du corps textile et
- comparer l'écart ($\Delta\mathbf{C}$) avec une différence entre la couleur secondaire ($\mathbf{C}$d) et la couleur typique ($\mathbf{C}$t).

**8.** Le procédé selon la revendication 7, la comparaison de l'écart (D$\mathbf{C}$) avec la couleur typique ($\mathbf{C}$t) comprenant les étapes suivantes

- calculer des composantes $\Delta\mathbf{C}1$ et $\Delta\mathbf{C}2$ de l'écart ($\Delta\mathbf{C}$) le long d'une différence entre la couleur secondaire ($\mathbf{C}$d) et la couleur typique ($\mathbf{C}$t) et perpendiculairement à celle-ci, et
- calculer un score d'erreur Q($\Delta\mathbf{C}1$, $\Delta\mathbf{C}2$) à partir des composantes D$\Delta\mathbf{C}1$ et $\Delta\mathbf{C}2$, qui dépend moins de $\Delta\mathbf{C}1$ que de $\Delta\mathbf{C}2$.

**9.** Le procédé selon l'une des revendications précédentes, comprenant l'étape consistant à émettre un avertissement et/ou à découper au moins une partie de la section lorsque la variabilité est supérieure à un deuxième niveau de variabilité.

**10.** Le procédé selon l'une des revendications précédentes, comprenant l'étape consistant à calculer la variabilité le long d'une direction donnée dans l'espace colorimétrique.

**11.** Le procédé selon la revendication 10, dans lequel la direction donnée est une composante principale ($w_n$) d'un ensemble de données comprenant une pluralité des paramètres de couleur mesurés (Cp) du corps.

**12.** Le procédé selon l'une des revendications précédentes, comprenant l'étape consistant à déterminer les composantes principales $w_n$ des valeurs d'échantillonnage $\mathbf{B}_m$.

**13.** Le procédé selon l'une des revendications précédentes, comprenant les étapes de
mesurer une pluralité de valeurs mesurées ($X_{km}$) à l'aide de la tête de capteur (12, 18, 24, 30, 44) et calculer, à partir des valeurs mesurées ($\mathbf{X}_{km}$), des valeurs d'échantillonnage $\mathbf{B}_m$ au moyen de transformations non linéaires ($F_k$), les transformations non linéaires ($F_k$) étant conçues pour rendre symétrique une distribution des valeurs d'échantillonnage $\mathbf{B}_m$, en particulier pour rendre cette distribution normale.

**14.** Le procédé selon l'une des revendications précédentes, comprenant l'étape consistant à utiliser la qualité évaluée pour afficher automatiquement un avertissement à un utilisateur.

**15.** Un procédé de fabrication d'un fil comprenant le procédé selon l'une des revendications précédentes.

**16.** Le procédé selon la revendication 15, comprenant les étapes de

comparer le paramètre de couleur ($C$p) dans la section avec une couleur de colorant ($C$d) qui indique la couleur d'un colorant avec lequel le corps textile allongé (52) doit être teint, et
teindre le fil avec le colorant ayant la couleur de colorant ($C$d).

**17.** Le procédé selon l'une des revendications précédentes 15 ou 16, comprenant l'étape consistant à retirer une section de fil en fonction de la qualité évaluée de la section.

**18.** Un dispositif pour la fabrication de fil, comprenant

au moins une tête de capteur (12, 18, 24, 30, 44) et
une unité de commande (31), **caractérisée en ce qu'**elle est conçue et structurée pour évaluer la qualité du fil en mettant en œuvre le procédé selon l'une des revendications précédentes.

**Fig. 1**

**Fig. 2**

**Fig. 3**

**Fig. 4**

Fig. 5

Fig. 6

Fig. 7

**EP 4 306 692 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H03234861 A
- US 20070013904 A1
- JP H0427851 A
- EP 3748343 A1
- EP 3751282 A1
- EP 3748342 A1
- CH 532526
- US 6650959 B